# EUROPÄISCHE PATENTSCHRIFT

(11) **EP 0 748 218 B1**
(45) Veröffentlichungstag und Bekanntmachung des Hinweises auf die Patenterteilung: **01.08.2001**
(21) Anmeldenummer: 95909746.0
(22) Anmeldetag: 20.02.1995
(51) Int. Cl.: A61K 31/135, A61K 9/70

(54) **TRANSDERMALES SYSTEM IN FORM EINES PFLASTERS MIT EINEM TAMOXIFEN-DERIVAT**
PLASTER-SHAPED TRANSDERMAL SYSTEM WITH A TAMOXIFEN DERIVATIVE
SYSTEME TRANSDERMIQUE SOUS FORME DE PANSEMENT COMPORTANT UN DERIVE DE TAMOXIFENE

(30) Priorität: 08.03.1994 DE 4407742
(43) Veröffentlichungstag der Anmeldung: 18.12.1996
(73) Patentinhaber: HEXAL AG, D-83607 Holzkirchen (DE)
(72) Erfinder: FISCHER, Wilfried, Dr., 83607 Holzkirchen (DE); KLOKKERS, Karin, Dr., 83607 Holzkirchen (DE)
(74) Vertreter: Boeters, Hans Dietrich, Dr.
(86) Internationale Anmeldenummer: EP9500603
(87) Internationale Veröffentlichungsnummer: WO9524187

(56) Entgegenhaltungen:
- EP-A- 0 240 131
- EP-A- 0 287 690
- WO-A-85/03228
- WO-A-93/12744
- GB-A- 2 273 873
- US-A- 4 198 435

## Beschreibung

Das wichtigste Medikament zur Behandlung spezieller Tumorformen, insbesondere hormonabhängiger Mammatumore, ist das Antioestrogen Tamoxifen (1-(p-3-Dimethylaminoethoxiphenyl)-trans-1,2-diphenyl-but-1-en). Tamoxifen ist in verschiedenen oralen Formulierungen unter verschiedenen Warenzeichen, insbesondere Novaldex^{R}, im Handel. Tamoxifen wird in der Langzeitbehandlung der Tumore peroral zwischen 10 und 40 mg pro Einzeldosis verabreicht. Die Tagesdosis liegt bei 20 bis 40 mg. In der Langzeittherapie zeigt Tamoxifen jedoch gravierende unerwünschte Nebenerscheinungen, so beispielsweise die paradoxe Stimulation der Ovarien. Dieses ist ein limitierender Faktor in der Langzeitverwendung von Tamoxifen. Während der Leberpassage nach Resorption aus dem Magen-Darm-Kanal entsteht aus dem Tamoxifen der wirksame Metabolit 4-Hydroxitamoxifen, der auf molekularer Basis etwa um den Faktor 100 stärker aktiv als Tamoxifen ist. Dieser Wert gründet sich auf die Publikation von Katzenellenbogen et al. (Bioactivities, Estrogen receptor interactions, and plasminogen activator-inducing activities of Tamoxifen and Hydroxytamoxifen Isomers in MCF-7 Human Brest Cancer Assays) in Cancer Research, 44 (1984) 112 - 119, zu MCF-7 Zellinien. Die zu 4-Hydroxitamoxifen isomere Substanz 3-Hydroxitamoxifen (Droloxifen) bindet ebenfalls in vitro sehr viel stärker an Estrogenrezeptoren, nämlich um den Faktor 20 bis 60, als Tamoxifen selbst. Ebenso wie beim 4-Hydroxitamoxifen ist die oestrogene Wirkkomponente geringer als beim Tamoxifen, während jeweils die antioestrogene Wirksamkeit höher liegt. Die oestrogene Komponente, sowohl von Tamoxifen als auch seinen Hydroxiderivaten, ist mit der stereoisomeren E-Form verbunden, während die antioestrogene Komponente durch die Z-Form hervorgerufen wird. Die Hydroxiderivate des Tamoxifens unterliegen allerdings nach oraler Applikation, wie sie beispielsweise nach DE-C-2 807 59.9 vorgeschlagen wird, einer extensiven Metabolisierung in der Leber. Die Substanzen werden sehr schnell konjugiert und damit inaktiviert (Rochefort et al. (Cellular and molecular mechanism of action of antioestrogens) in J. Steroid Biochem., 19 (1983) 69 - 74).

Aufgrund der hohen Rezeptoraffinität der Hydroxitamoxifenderivate wäre der therapeutische Einsatz wünschenswert, wird jedoch durch die perorale Route aufgrund der schnellen Inaktivierung in der Leber sehr stark limitiert. Aus diesem Grunde beschreiben die EP-B-0 169 214 und EP-B-0 151 326 die topische Anwendung von 4-Hydroxitamoxifen in einem hydroalkoholischen Gel. Dieses Gel ist für die topische Applikation von 4-Hydroxitamoxifen vorgesehen. Mauvais-Jarvis et al. in Cancer Research, 46 (1986) 1521-1525 beschreiben die topische Anwendung einer alkoholischen Lösung bei 12 Patientinnen. In dieser Arbeit wurde die Verteilung radioaktiven 4-Hydroxitamoxifens in das Brustdrüsengewebe untersucht. Die Autoren fanden, daß die perkutane topische Applikation von 4-Hydroxitamoxifen zuerst zu einer hohen Retention des Wirkstoffes im estrogenrezeptorhaltigen Brustdrüsengewebe führte und der Übergang in das Plasmakompartiment sehr lange verzögert war. Nur etwa 0,5 % der applizierten Dosis wurde unverändert im Plasma wiedergefunden.

Nun ist es aber wünschenswert, im Falle von beispielsweise metastasierenden Tumoren, bei denen 4-Hydroxitamoxifen nicht jeweils topisch appliziert werden kann, eine systemische Applikationsart zur Verfügung zu stellen. Nachteilig ist die in oben genannten EP-B-Veröffentlichungen beschriebene perkutane Arzneiform, und zwar das hydroalkoholische Gel. Solche Gele können per se nicht auf definierte Hautareale aufgetragen werden und sind während der Einwirkzeit des Geles nicht vor äußeren Einflüssen, wie Abwaschen oder Abreiben an der Kleidung, geschützt. Die Zufuhr von Wirkstoff aus solchen Gelen ist dementsprechend sehr variabel und unsicher.

Aufgabe der Erfindung ist es, für Tamoxifen-Derivate eine systemische Applikationsart zur Verfügung zu stellen.

Die der Erfindung zugrundeliegende Aufgabe wird nun durch ein transdermales System in Form eines Pflasters gelöst, das 3-Hydroxytamoxifen und/oder 4-Hydroxytamoxifen in Form einer Lösung in einem Allcohol als resorptionsförderndes Additiv umfaßt. Das Tamoxifen-Derivat wird also in Form eines Wirkstoffpflasters, eines sogenannten transdermales Systems, für die systemische Zufuhr vorgesehen. Da 3-Hydroxytamoxifen oder 4-Hydroxytamoxifen sehr lipophil sind, können sie ohne Zuschlagsstoffe bzw. Additive nicht die lipophile Barriere des Stratum Corneum der Haut überwinden. Um eine ausreichende systemische Zufuhr von Tamoxifen-Derivaten vorzusehen, wird daher erfindungsgemäß mindestens ein resorptionsförderndes Additiv hinzugefügt.

So wird das gewünschte Tamoxifen-Derivat erfindungsgemäß in Form einer Lösung in einem Alkohol vorgesehen, wobei man den Alkohol auch als Wasser/Alkohol-Gemisch einsetzen kann. Das Tamoxifen-Derivat liegt dann also in Form einer alkoholischen oder hydroalkoholischen Lösung vor, wobei es sich bei dem Alkohol um ein niedrig siedendes Alkanol mit einer Kettenlänge von 1 bis 8 Kohlenstoffatomen, beispielsweise um Ethanol, oder um einen aromatischen Alkohol, beispiesweise Benzylakohol, handeln kann. Um die Hautpermeation gegenüber alkoholischen Lösungen bzw. von alkoholischen Lösungen weiter zu erhöhen, was dann erwünscht ist, wenn man möglichst kleine, praxisgerechte Transdermalsysteme anbieten möchte, enthält das transdermale System ferner einen Gehalt an Vitamin E oder an einem Vitamin E-Derivat. Erfindungsgemäß wurde überraschenderweise festgestellt, daß durch Zusatz von natürlichem Vitamin E (Copherol^{R}) die Hautpermeation weiter gesteigert werden kann. Dieses Ergebnis ist umso überraschender, als der Zusatz von Ölsäure, 1,8-Cineol oder Phospholipiden in Mengen, die mit denen von Vitamin E vergleichbar sind, eher zu einer Abnahme der Hautpermeation in Vergleich zu reinem Ethanol führt. Dabei sind Ölsäure, 1,8-Cineol und Phospholipid als sehr wirsame Resorptionsförderer für beispielsweise Sexualhormone bekannt.

Erfindungsgemäß liegt das Tamoxifen-Derivat im transdermalen System vorzugsweise als gesättigte Lösung vor.

Gemäß der Ausführungsform der Erfindung wird ein transdermales System vorgesehen, das ein Pflaster mit einem Reservoir für die Lösung des Tamoxifen-Derivats ist (Pflaster vom Reservoir-Typ). Dieses erfindungsgemäße Pflaster kann
(a) eine lösungsundurchlässige Abdeckschickt (Backing Foil),
(b) ein schichtartiges Element mit Hohlraum,
(c) eine microporöse oder semipermeable Membran,
(d) eine selbstklebende Schicht (Haftschicht) und
(e) gegebenenfalls eine abziehbare Abdeckfolie aufweisen.

Dabei kann das schichtartige Element mit Hohlraum durch die Abdeckschicht und die Membran gebildet werden.

Alternativ kann das erfindungsgemäße Pflaster
(a) eine lösungsundurchlässige Abdeckschicht (Backing Foil),
(b) einen offenporigen Schaum, einen geschlossenporigen Schaum, eine gewebeartige Schicht oder eine vliesartige Schicht als Reservoir,
(c) sofern die Schicht gemäß (b) nicht selbstklebend ist, eine selbstklebende Schicht (Haftschicht) und
(d) gegebenenfalls eine abziehbare Abdeckfolie aufweisen.

Es versteht sich von selbst, daß die selbstklebenden Schichten mit der Wirkstofflösung kompatibel sein müssen.

Erfindungsgemäß kann die Abdeckschicht (Backing Foil) aus Polyester, Polypropylen oder Polyethylen gebildet sein, wobei die Abeckschicht (Backing Foil) eine Dicke im Bereich von 4 bis 60 und insbesondere 5 bis 50 µm besitzen kann.

Erfindungsgemäß kann die mikroporöse Membran die Geschwindigkeit, mit der die Wirkstofflösung an die Haut abgegeben wird, in unterschiedlichem Maße kontrollieren. Dabei kann die Porengröße so gestaltet sein, daß die Wirkstofflösung lediglich durch Oberflächenspannung am Ausfließen aus dem Reservoir gehindert, aber keine Freisetzungskontrolle bewirkt wird. Sie kann aber auch mit solch kleinen Poren versehen sein, daß eine gezielte Beeinflussung der Diffusionsgeschwindigkeit der Lösung aus dem Reservoir hervorgerufen wird.

Erfindungsgemäß kann die Membran aus einem inerten Polymeren bestehen, insbesondere Polypropylen, Polyvinylacetat oder Silikon.

Mit den genannten selbstklebenden Schichten kann das gesamte therapeutische System auf die Haut aufgeklebt werden.

Nachstehend wird die Erfindung durch Beispiele näher erläutert.

### Beispiel 1

Eine gesättigte Lösung von 4-Hydroxitamoxifen in 96-proz. Ethylalkohol wird in einer modifizierten Franzzelle auf die exzidierte Haut von Nacktmäusen gegeben. Es wird kontinuierlich die. durch 2,5 cm² permeierende Menge Wirkstoff in das Akzeptormedium (Phosphatpuffer pH = 5,5) mittels HPLC gemessen. Die in 48 Stunden permeierte Menge beträgt ca. 400 µg.

### Beispiel 2

In oben beschriebener modifizierter Franzzelle wird eine gesättigte Lösung von 4-Hydroxitamoxifen in Benzylalkohol untersucht. Die in 48 Stunden permeierte Substanzmenge beträgt ebenfalls etwa 400 µg.

### Beispiel 3

In der Diffusionsapparatur gemäß Beispiel 1 wird eine gesättigte Lösung von 4-Hydroxitamoxifen in einem Gemisch von Ölsäure (10 %) und Ethanol (90 %) untersucht. Die in 48 Stunden permeierte Substanzmenge beträgt etwa 150 µg.

### Beispiel 4

Es wird eine gesättigte Lösung von 4-Hydroxitamoxifen in einem Gemisch aus Phospholipid (Phospholipon 80; 25 %) und Ethanol (75 %) untersucht. Die in 48 Stunden permeierte Substanzmenge beträgt ca. 40 µg.

### Beispiel 5

Eine gesättigte Lösung von 4-Hydroxitamoxifen in einem Gemisch aus natürlichem Vitamin E (Copherol F 1300; 10 %) und Ethanol (90 %) wird in der Diffusionszelle nach Beispiel 1 untersucht. Die in 48 Stunden diffundierte Substanzmenge beträgt ca. 1200 µg.

### Beispiel 6

Ein transdermales therapeutisches System vom Reservoirtyp, enthaltend eine gesättigte Lösung von 4-Hydroxitamoxifen in 90 % Ethanol und 10 % Vitamin E, welches durch eine microporöse Membran, enthaltend 28 % EVA (Typ MSX1154P), und eine Schicht eines selbstklebenden Haftklebers (Typ Cotran Nr. 9871) (beide 3M Medica, D-Borken) zur Hautseite abgeschlossen wurde, zeigt in der Franzdiffusionszelle gemäß Beispiel 1 an der Haut nackter Mäuse eine in 48 Stunden permeierte Hydroxitamoxifenmenge von etwa 350 µg. In diesem Fall stellt die microporöse Membran ein Steuerelement dar, da die pro Zeit permeierte Substanzmenge etwa um den Faktor 2 niedriger liegt als die bei der Haut selbst.

### Beispiel 7

Es wurden mehrere Versuche mit einem östrogen-abhängigen Mamma-Human-Tumor vom Typ MAXF NCF7/10 durchgeführt, den man an Nacktmäusen anwachsen ließ. Als Wirkstoff diente z-4-Hydroxytamoxifen. Einzelheiten sind der folgenden Tabelle zu entnehmen.

**Versuch 1:** Kontrolle ohne z-4-Hydroxytamoxifen-Verabreichung.

**Versuche 2 und 3** (Vergleiche): Bei diesen Versuchen wurde der Wirkstoff in Olivenöl subcutan appliziert.

**Versuche 4 und 5** (Erfindung): Für diese Versuche wurden Pflaster (TTS vom Reservoirtyp) gemäß Beispiel 6 verwendet, bei denen der Wirkstoff in 96-proz. Ethanol unter Zusatz von 10 % Vitamin E (Copherol F1300) vorlag. Bei Versuch 4 wurde das Pflaster transdermal im Nackenbereich der Versuchstiere und bei Versuch 5 topisch auf den Tumor aufgebracht.

**Versuch 6** (Vergleich): Bei diesem Versuch wurde der Wirkstoff peroral verabreicht.

Die perorale Wirkstoffgabe stellt zur Zeit die Standardtherapie bei Mammakarzinomen dar. Eine Gegenüberstellung von Versuch 6 mit Versuchen 2 bis 5 zeigt jedoch, daß die perorale Applikation allen anderen Applikationen unterlegen ist, wobei zu berücksichtigen ist, daß bei Versuch 6 die 100-fache Dosis gegenüber Versuchen 3 bis 5 und die 10-fache Dosis gegenüber Versuch 2 eingesetzt wurde.

Eine Gegenüberstellung der Versuche 3 und 4 zeigt ferner, daß die transdermale Gabe (Versuch 4) der subkutanen Gabe (Versuch 3) ebenbürtig ist. Beide Versuche zeigen (bezogen auf Dosis/kg Körpergewicht) eine etwa 100-fach stärkere Wirksamkeit als die perorale Wirkstoffgabe. Schließlich zeigt eine Gegenüberstellung der Versuche 3 und 5, daß die topische Applikation eine etwa 10-fach stärkere Wirksamkeit als die subkutane Gabe liefert.

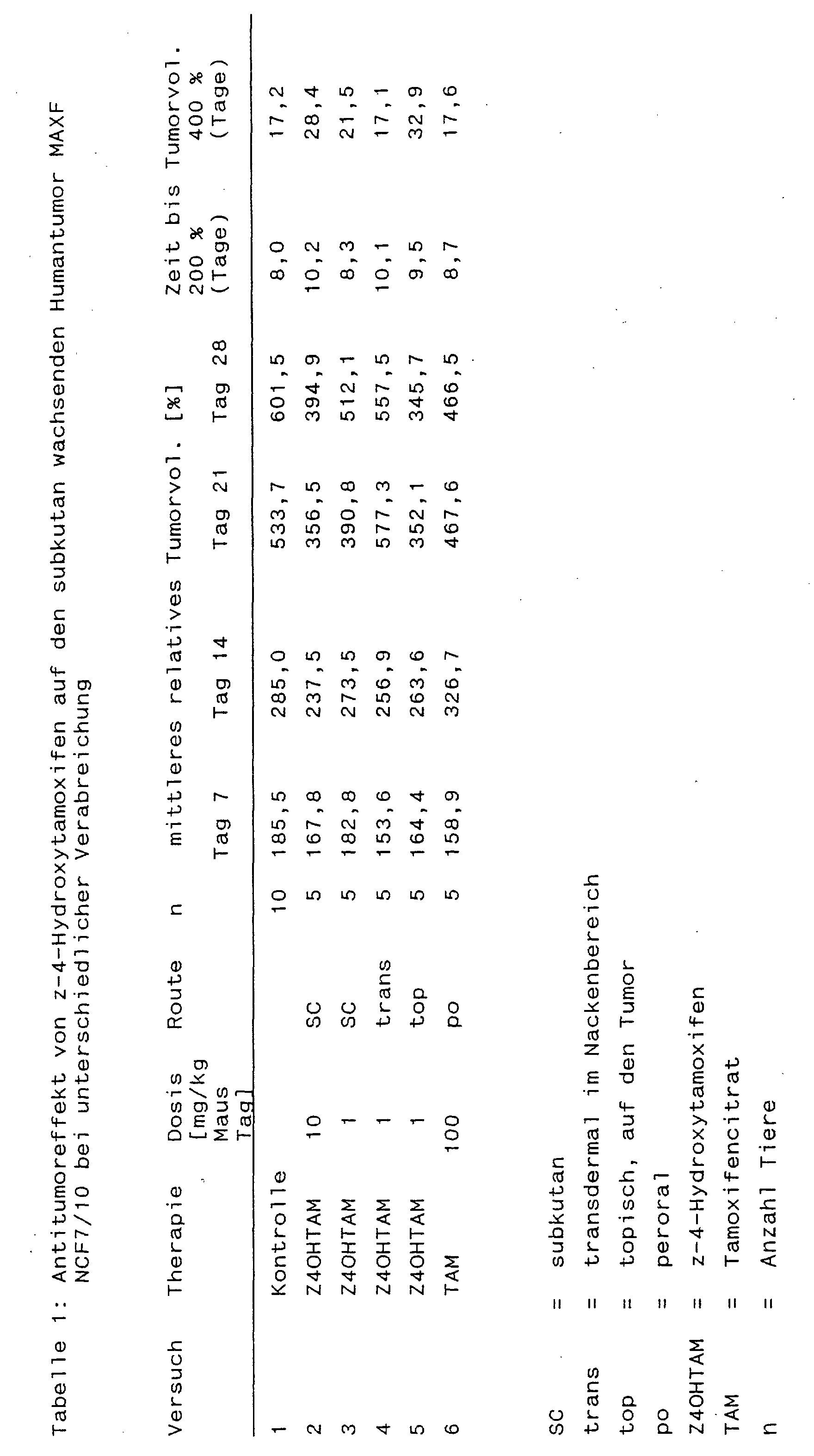

## Patentansprüche

1. Transdermales System in Form eines Pflaster, **gekennzeichnet** durch 3-Hydroxytamoxifen und/oder 4-Hydroxytamoxifen als Tamoxifen-Derivat (Wirkstoff), wobei
(i) das Tamoxifen-Derivat in Form einer Lösung in einem Alkohol als resorptionsförderndes Additiv vorliegt,
(ii) das transdermale System einen Gehalt an Vitamin E oder an einem Vitamin E-Derivat aufweist,
(iii) ein Pflaster mit einem Reservoir für die Lösung des Tamoxifen-Derivats ist (Pflaster vom Reservoir-Typ) und
(iv) aufweist:
(a) eine lösungsundurchlässige Abdeckschicht (Backing Foil),
(b) ein schichtartiges Element mit Hohlraum,
(c) eine microporöse oder semipermeable Membran,
(d) eine selbstklebende Schicht (Haftschicht) und
(e) gegebenenfalls eine abziehbare Abdeckfolie.

2. Transdermales System nach Anspruch 1, dadurch **gekennzeichnet**, daß das schichtartige Element mit Hohlraum durch die Abdeckschicht und die Membran gebildet wird.

3. Transdermales System nach Anspruch 1 oder 2, dadurch **gekennzeichnet,** daß die Abdeckschicht (Backing Foil) aus Polyester, Polypropylen oder Polyethylen gebildet ist.

4. Transdermales System nach Anspruch 3, **gekennzeichnet** durch eine Abdeckschicht (Backing Foil) mit einer Dicke im Bereich von 4 bis 60 und insbesondere 5 bis 50 *µ*m.

5. Transdermales System nach Anspruch 1, dadurch **gekennzeichnet,** daß die Membran aus einem inerten Polymeren besteht, insbesondere Polypropylen, Polyvinylacetat oder Silikon.

6. Transdermales System nach einem der vorhergehenden Ansprüche, dadurch **gekennzeichnet**, daß der Alkohol als Wasser/Alkohol-Gemisch vorliegt.

7. Transdermales System nach einem der vorhergehenden Ansprüche, **gekennzeichnet** durch ein niedrigsiedendes Alkanol, insbesondere ein C₁₋₈-Alkanol, wie Ethanol, oder einen aromatischen Alkohol, wie Benzylalkohol, als resorptionsförderndes Additiv.

8. Transdermales System nach einem der vorhergehenden Ansprüche, dadurch **gekennzeichnet,** daß das Tamoxifen-Derivat als gesättigte Lösung vorliegt.

## Claims

1. Transdermal system in the form of a patch, characterised by 3-hydroxytamoxifen and/or 4-hydroxytamoxifen as tamoxifen derivative (active ingredient), wherein
(i) the tamoxifen derivative is in the form of a solution in an alcohol as the absorption-promoting additive,
(ii) the transdermal system is provided with a content of vitamin E or of a vitamin E derivative,
(iii) is a patch having a reservoir for the solution of the tamoxifen derivative (reservoir-type patch), and
(iv) is provided with:
(a) a solution-impermeable backing foil,
(b) a layer-like element having a cavity,
(c) a microporous or semi-permeable membrane,
(d) a self-adhesive layer (adhesive layer) and
(e) optionally a removable backing film.

2. Transdermal system according to claim 1, characterised in that the layer-like element having a cavity is formed by the backing foil and the membrane.

3. Transdermal system according to claim 1 or 2, characterised in that the backing foil is formed from polyester, polypropylene or polyethylene.

4. Transdermal system according to claim 3, characterised by a backing foil having a thickness in the range from 4 to 60 *µ*m, and especially from 5 to 50 *µ*m.

5. Transdermal system according to claim 1, characterised in that the membrane consists of an inert polymer, especially polypropylene, polyvinyl acetate or silicone.

6. Transdermal system according to any one of the preceding claims, characterised in that the alcohol is in the form of a water/alcohol mixture.

7. Transdermal system according to any one of the preceding claims, characterised by a low-boiling point alkanol, especially a C₁₋₈-alkanol, such as ethanol, or an aromatic alcohol, such as benzyl alcohol, as absorption-promoting additive.

8. Transdermal system according to any one of the preceding claims, characterised in that the tamoxifen derivative is in the form of a saturated solution.

## Revendications

1. Système transdermique sous forme d'un pansement, caractérisé par le 3-hydroxytamoxifène et/ou le 4-hydroxytamoxifène en tant que dérivé de tamoxifène (substance active), dans lequel
(i) le dérivé de tamoxifène est présent sous forme d'une solution dans un alcool en tant qu'additif favorisant la résorption,
(ii) le système transdermique comprend une teneur en vitamine E ou en un dérivé de vitamine E,
(iii) est un pansement avec un réservoir pour la solution de dérivé du tamoxifène (pansement du type à réservoir) et
(iv) comprend :
(a) une couche de recouvrement imperméable à la solu-tion (backing foil),
(b) un composant de type stratifié avec cavité,
(c) une membrane microporeuse ou semi-perméable,
(d) une couche auto-adhésive (couche d'adhérence) et
(e) éventuellement une feuille de recouvrement détachable.

2. Système transdermique selon la revendication 1, caractérisé en ce que le composant de type stratifié avec cavité est formé par la couche de recouvrement et la membrane.

3. Système transdermique selon la revendication 1 ou 2, caractérisé en ce que la couche de recouvrement (backing foil) est formé de polyester, polypropylène ou polyéthylène.

4. Système transdermique selon la revendication 3 ou 2, caractérisé par une couche de recouvrement (backing foil) ayant une épaisseur dans la plage de 4 à 60 µm et en particulier de 5 à 50 µm.

5. Système transdermique selon la revendication 1, caractérisé en ce que la membrane est constituée d'un polymère inerte; en particulier, le polypropylène, le poly(acétate de vinyle) ou la silicone.

6. Système transdermique selon l'une quelconque des revendications précédentes, caractérisé en ce que l'alcool est présent sous forme de mélange eau-alcool.

7. Système transdermique selon l'une quelconque des revendications précédentes, caractérisé par un alcool à faible point d'ébullition, en particulier un alcool en C₁ à C₈, comme l'éthanol, ou un alcool aromatique, comme l'alcool benzylique, en tant qu'additif favorisant la résorption.

8. Système transdermique selon l'une quelconque des revendications précédentes, caractérisé en ce que le dérivé de tamoxifène est présent sous la forme de solu-tion saturée.
